# EUROPÄISCHE PATENTSCHRIFT

(11) **EP 0 896 000 B1**
(45) Veröffentlichungstag und Bekanntmachung des Hinweises auf die Patenterteilung: **03.03.2004**
(21) Anmeldenummer: 98113455.4
(22) Anmeldetag: 18.07.1998
(51) Int. Cl.: C07K 5/02

(54) **Verfahren zur Isolierung von 1-[N2-((S-Ethoxycarbonyl)-3-phenylpropyl)-N6-trifluoracetyl]-L-lysyl-L-prolin (Lisinopril(Tfa)ethylester, LPE)**
Process for isolation of 1-[N2-((S-Ethoxycarbonyl)-3-phenylpropyl)-N6-trifluoroacetyl]-L-lysyl-L-proline (Lisinopril (Tfa) ethylester, LPE)
Procédé pour l'isolement de l' 1-[N2-((S-éthoxycarbonyle)-3-phénylpropyle)-N6-trifluoroacétyl]-L-lsysl-L-proline (Lisinopril (Tfa) éthyléster, LPE)

(30) Priorität: 30.07.1997 DE 19732839
(43) Veröffentlichungstag der Anmeldung: 10.02.1999
(73) Patentinhaber: Degussa AG, 40474 Düsseldorf (DE)
(72) Erfinder: Kottenhahn, Matthias, Dr., 63579 Freigericht (DE); Möller, Roland, 63546 Hammersbach (DE); Kraft, Michael, 63517 Rodenbach (DE); Drauz, Karlheinz, Prof. Dr., 63579 Freigericht (DE); Stingl, Klaus, Dr., 63755 Alzenau (DE)

(56) Entgegenhaltungen:
- EP-A- 0 523 449
- EP-A- 0 645 398
- DE-A- 4 331 540
- CHEMICAL ABSTRACTS, vol. 126, no. 4, 27. Januar 1997 Columbus, Ohio, US; abstract no. 47560, M KURAUCHI ET AL.: "Method for producing dipeptide derivative as intermediate for antihypertensive agent" XP002083885 & JP 08 283289 A (AJINOMOTO KK) 29. Oktober 1996

## Beschreibung

Die Erfindung betrifft ein Verfahren zur Isolierung von 1-[N²-((S)-Ethoxycarbonyl)-3-phenylpropyl)-N⁶-trifluoracetyl]-L-lysyl-L-prolin (LPE, Verbindung I).

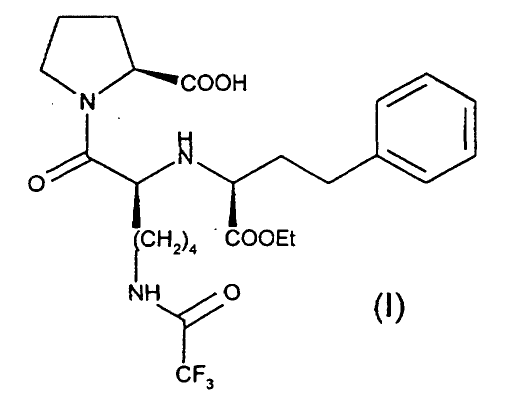

N-substituierte Aminosäuren dieses Typs sind wertvolle Zwischenprodukte für die Herstellung von Inhibitoren des Angiotensin Converting Enzymes (ACE), die als Blutdruckregulatoren wirken. (I) ist das direkte Vorprodukt für 1-[N²-((S)-Carboxy)-3-phenylpropyl)]-L-lysyl-L-prolin (Lisinopril II), welches hervorragende Therapieresultate bei der Bluthochdruckbekämpfung aufweist (Zestril®, Coric®, Prinivil®).

Verbindung (I) erhält man nach dem Stand der Technik durch reduktive Aminierung von 2-Oxo-4-phenylbuttersäureethylester mit dem Dipeptid Lys(Tfa)-Pro.

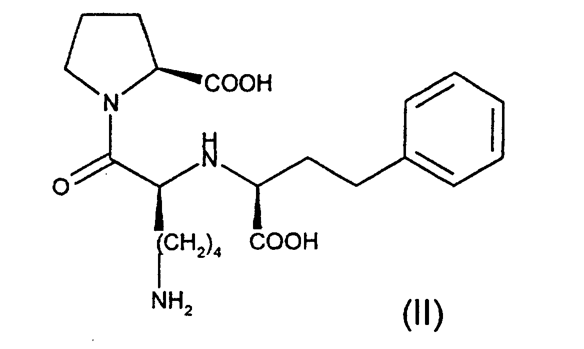

Im J. Org. Chem. 1988, 53,836 - 844 wird ein solches Verfahren beschrieben. (I) wird danach in einer Ausbeute von 42 % durch basische Extraktion der Reaktionsrohlösung, eine nachgeschaltete Extraktion des Produktes in Methylenchlorid bei pH 4,6 und anschließende Kristallisation nach Lösungsmittelwechsel aus Methyl-tert.-butylether, Cyclohexan erhalten.

Die EP 05 23 449 betrifft die Synthese von (I), das gemäß Beispiel 3 mit einer Ausbeute von 60 % gewonnen wird. Die Aufarbeitung der nach diesem Verfahren erhaltenen Reaktionsrohlösung beinhaltet neben dem basischen und einem sauren Extraktionsschritt mit 1,1,1-Trichlorethan eine Kristallisation aus Methyl-tert.-butylether.

Grundsätzlich sind auch weitere, nicht auf reduktiver Aminierung beruhende, jedoch weniger vorteilhafte Verfahren zur Herstellung von Verbindung (I) bekannt (EP 0 336 368 A2). Hierin wird die wäßrige Produktphase mit Methylenchlorid extrahiert. Nach Trocknung der organischen Phase über Natriumsulfat wird aber wiederum das Lösungsmittel zur Kristallisation in Methyl-tert.-butylether gewechselt.

Die Kristallisation aus reinem Methyl-tert.-butylether führt zu einem schlecht filtrierbaren Kristallkorn und zu oftmals ungenügenden Ausbeuten (EP 0 645 398 A1). Läßt man (I) aus Lösungen mit hoher Konzentration kristallisieren, wird eine zusätzliche Umkristallisation notwendig. Beschrieben ist auch der Zusatz von Cyclohexan (J. Org. Chem., 1988, 53, 836 - 844) bei der Kristallisation zur Ausbeuteerhöhung. Dabei besteht jedoch die Gefahr der Abscheidung als Öl, was eine Isolierung des Produkts nicht nur im technischen Maßstab stark erschwert.

In EP 0 645 398 A1 wird eingehend die Möglichkeit der Kristallisation von Verbindung (I) aus verschiedenen Lösungsmitteln oder Lösungsmittelgemischen geprüft. Dabei stellte sich heraus, daß beim Einsatz von Methyl-tert.-butylether oder Methyl-tert.-butyletherhaltigen Gemischen der Restlösungsmittelgehalt der Kristalle nach der Kristallisation sehr groß war, bzw. Restlösungsmittel im Kristall eingebunden ist. Das so erhaltene Rohmaterial an LPE ist extrem schwer zu trocknen. Lange Trocknungszeiten, die die Produktqualität beeinträchtigen können (DKP-Bildung speziell bei erhöhten Temperaturen), bzw. die Neigung zu Verklebungen des Produkts, machen spezielle aufwendige Trocknungskonzepte notwendig.

Aus WO 95/07928 ist eine Aufarbeitungsart bekannt, die eine Extraktion mit anschließender Kristallisation beschreibt. Hierbei wird in einem pH-Bereich von 0 - 6,3 das Rohmaterial der LPE-Herstellung ggf. mittels mehrerer flüssig/flüssig-Extraktionsschritte vorgereinigt, bevor es aus einem Gemisch von Methyl-tert.-butylether und Methylcyclohexan bei erniedrigten Temperaturen kristallisiert wird. Auch hier fand zwischen der Extraktion und der Kristallisation ein Lösungsmitteltausch statt.

Nachteilig an den Verfahren zur Aufarbeitung von LPE des Standes der Technik ist, daß häufig umweltgefährdende chlorierte Lösungsmittel eingesetzt werden und während der Aufarbeitung das Lösungsmittel gewechselt werden muß, was im technischen Maßstab schwierig vollständig erreicht werden kann und wodurch nur unzureichend definierte Lösungsmittelzusammensetzungen für die Kristallisation eingestellt werden können. Darüber hinaus sind für solche Lösungsmittelwechsel aufgrund der Empfindlichkeit des Produktes nur milde Temperaturbedingungen erlaubt, was lange Destillationszeiten mit sich bringt. Die Verfahren des Standes der Technik führen zudem oftmals zu schlecht filtrierbarer, mit viel Restlösemittel inkludierten Kristallen. Ein solches Produkt benötigt lange Trocknungsprozedere, die im technischen Maßstab Verbackungen und Verklebungen nur schwierig kontrollierbar machen.

Angesichts des hierin angegebenen und diskutierten Standes der Technik war es mithin Aufgabe der Erfindung, ein neues Verfahren zur Isolierung von LPE (I) zu finden, das es erlaubt, über eine vereinfachte und wirtschaftlichere Verfahrensweise das aus einem LPE-Herstellprozeß erhaltene Rohmaterial aus einer wäßrigen Produktphase besser isolieren zu können, welches seinerseits durch günstigere Kristalleigenschaften des Fällungsmaterials die üblichen langen, das LPE (I) stressenden Trocknungszeiten verringern hilft.

Aufgabe der Erfindung war es weiterhin, mit den neuen einfacheren Isolierverfahren ein LPE-Endmaterial zu generieren, das bei vergleichbaren Trocknungszeiten besonders bezüglich des Restlösemittelgehaltes besser als das des Standes der Technik ist.

Aufgabe der Erfindung war auch ein verbessertes LPE (I).

Verfahrenstechnisch werden diese Aufgaben durch ein Verfahren mit den Merkmalen des kennzeichnenden Teils des Anspruchs 1 gelöst. Vorteilhafte Verfahrensmodifikationen der Erfindung sind Gegenstand der auf Anspruch 1 rückbezogenen abhängigen Unteransprüche. Im Hinblick auf das Produkt zeigt Anspruch 12 eine Lösung auf.

Dadurch, daß man aus einer nach dem Verfahren des Standes der Technik hergestellten wäßrigen Produktlösung eines LPE-Herstellprozesses das LPE (I) mit einem Lösungsmittel oder Lösungsmittelgemisch extrahiert und anschließend dieses Lösungsmittel oder Lösungsmittelgemisch als ein Hauptbestandteil des Lösungsmittels oder Lösungsmittelgemisches einsetzt, aus welchem das LPE (I) kristallisiert wird, gelangt man mittels dieses vereinfachten und sehr viel wirtschaftlicheren Verfahrens zu Kristallen aus LPE, die sich durch günstigere Kristalleigenschaften auszeichnen. Das so generierte LPE weist nach der Kristallisation nur geringe Lösungsmitteleinschlüsse auf und besitzt aufgrund seiner gut ausgebildeten Kristallstruktur eine hervorragende Filtrierbarkeit. Der geringe Gehalt an inkludiertem Restlösemittelanteil ist mit ursächlich dafür, daß die vormals langen Trocknungszeiten pro Trocknungsansatz deutlich reduziert werden können. Dabei besitzt das so hergestellte LPE einen ebenso hervorragend geringen Nebenproduktgehalt wie das des Standes der Technik. Somit kann überraschenderweise trotz der vereinfachten Verfahrensweise zur Extraktion und Kristallisation von LPE ein gegenüber dem Stand der Technik vorteilhafteres Produkt hergestellt werden, was mit ursächlich dafür ist, ein kostengünstigeres Verfahren zur LPE-Herstellung bereitzustellen.

Besonders vorteilhaft ist es, wenn das neue Verfahren zur Extraktion und Kristallisation von LPE (I) mit Lösungsmitteln oder Lösungsmittelgemischen bestehend aus Estern und/oder Ketonen der allgemeinen Formel (III) 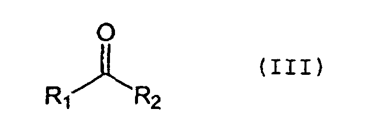 durchgeführt wird, wobei ggf. diese Lösungsmittel oder Lösungsmittelgemische mit offenkettigen aliphatischen oder cycloaliphatischen Kohlenwasserstoffen als Lösungsmittel zusätzlich vermischt werden.

Die Reste R₁ und R₂ stehen dabei vorteilhafterweise für eine Gruppe aus (C₁-C₆)-Alkylresten. Die Reste können dabei linear oder verzweigt sein. Insbesondere können die Reste beinhalten: Methyl, Ethyl, n-Propyl, iso-Propyl, n-Butyl, sec-Butyl, iso-Butyl, tert.-Butyl, n-Pentyl, n-Hexyl. Der Rest R₂ umfaßt die Gruppe des Restes R₁ sowie die Gruppe der (C₁-C₆)-Alkoxyreste. Diese können linear oder verzweigt sein. Insbesondere sind für R₂ zulässig: Methoxy, Ethoxy, n-Propoxy, iso-Propoxy, n-Butoxy, sec-Butoxy, iso-Butoxy, tert.-Butoxy, n-Pentoxy, n-Hexoxy. Dabei kann die Vermischung des Lösungsmittels oder Lösungsmittelgemisches mit den Kohlenwasserstoffen vor oder auch nach der Extraktion durchgeführt werden. Als offenkettige aliphatische Kohlenwasserstoffe kommen solche in Betracht, welche 5-9 C-Atome enthalten. Diese können linear oder beliebig verzweigt sein. Mit cycloaliphatischen Kohlenwasserstoffen sind Ringe der Größe 5 bis 7 gemeint, welche beliebig mit (C₁-C₄)-Alkylresten, welche wiederum verzweigt vorliegen können, substituiert sein können. Als ganz besonders vorteilhafte Lösungsmittel bzw. Lösungsmittelgemische haben sich erwiesen:
- Ester -: Ethylacetat, n-Propylacetat, n-Butylacetat, Ethylpropionat
- Ketone -: Methyl-isobutylketon, Diethylketon, Methylisopropylketon
- Aliphate -: n-Pentan, n-Hexan, Cyclohexan, Methylcyclohexan

Ebenfalls eignen sich auch höhere Homologe der oben beschriebenen Lösungsmittel, wobei sich eine natürliche Grenze aufgrund der steigenden Siedepunkte und somit einer Verschlechterung der Trocknungseigenschaften des feuchten Kristallisats ergibt. Als besonders vorteilhafte Lösungsmittelgemische hat sich jedwede Kombination der oben genannten besonders vorteilhaften Lösungsmittel erwiesen.

Diese Lösungsmittel und/oder Lösungsmittelgemische können vorteilhafterweise ebenfalls zur Vorreinigung der wäßrigen Produktphase bei einem pH zwischen 0 und 3,5 eingesetzt werden. Diese Vorreinigung erfolgt vor der eigentlichen Extraktion des LPEs (I) in die organische Phase. Sie führt dazu, daß die wäßrige Produktphase an Nebenprodukten verarmt. Dadurch, daß man die gleichen Lösungsmittel und/oder Lösungsmittelgemische einsetzen kann, welche ebenfalls zur Extraktion und Kristallisation benutzt werden können, vermindert sich vorteilhafterweise die Notwendigkeit der Bereitstellung zusätzlicher Lagerkapazität für Lösungsmittel und/oder Lösungsmittelgemische, welche sich von denen der Extraktion und Kristallisation des LPEs (I) unterscheiden. Ebenfalls wird dadurch die Möglichkeit der Recyclierung der Lösungsmittel deutlich verbessert. Bei einem technischen Prozeß ist es mithin immer besonders vorteilhaft, so wenig unterschiedliche Lösungsmittel wie möglich einzusetzen.

Zudem ist eine vorteilhafte Ausgestaltung des Verfahrens darin zu sehen, daß nach der oben beschriebenen Lösungsmittelbehandlung aber vor der eigentlichen Extraktion des LPEs (I) in ein organisches Lösungsmittel oder Lösungsmittelgemisch eine Aktivkohleklärung im gleichen pH-Bereich von 0-3,5 durchgeführt wird. Dadurch wird die Kristallisierbarkeit des LPEs (I) nochmals deutlich verbessert.

Die oben angesprochene Extraktion des LPEs (I) aus seiner wäßrigen Produktphase wird anschließend in einem pH-Bereich zwischen 3,5 und 6,3, besonders bevorzugt ist 3,9 bis 4,8, durchgeführt. Ganz besonders bevorzugt ist, daß die so erhaltene organische Lösung des LPEs (I) vor der Kristallisation mit Wasser bei einem pH-Wert von 4,8 bis 6,3, besonders bevorzugt ist der Bereich von 5,7 bis 6,0, ausgewaschen wird und man die wäßrige Phase von der organischen abtrennt.

Erfindungsgemäß kann die LPE-Extraktionslösung vor der abschließenden Kristallisation ggf. durch Destillation azeotrop entwässert werden. Die angegebenen Lösungsmittel bzw. Lösungsmittelgemische fungieren dabei als wasserschleppendes Medium.

Die oben angesprochenen Extraktionsschritte werden vorteilhafterweise bei einer Temperatur zwischen 0 °C und 60 °C durchgeführt, bevorzugt sind 20 °C bis 50 °C, besonders bevorzugt sind 35 °C bis 45 °C. Dabei benutzt man bei Anwendung von Lösungsmittelgemischen Volumenverhältnisse zwischen Ester und/oder Ketonen und dem eingesetzten aliphatischen/cycloaliphatischen Kohlenwasserstoffen zwischen 1 : 0,01 und 1 : 100, ganz besonders vorteilhaft ist ein Verhältnis von 1 : 0,5 bis 1 : 2. Die abschließende Kristallisation erfolgt erfindungsgemäß bei Temperaturen zwischen -40 °C und +50 °C. Nach der Kristallisation kann vorteilhafterweise nochmals ein aliphatischer bzw. cycloaliphatischer Kohlenwasserstoff, wie er oben schon näher beschrieben wurde, ggf. nach Einengen der Mutterlauge, zu dieser hinzugefügt werden. Dadurch kommt es zu einer erneuten Kristallisation, wobei die vormals schon hohen Ausbeuten von >75 % an LPE (I) nochmals um ca. 10 % gesteigert werden können.

Die vorliegende Erfindung umfaßt auch ein neues LPE (I), welches sich durch eine neue und vorteilhafte Kristallmodifikation auszeichnet. LPE (I), das nach dem Stand der Technik hergestellt wurde, zeigt ein komplett anderes Röntgenbeugungsverhalten als eines, welches nach der vorliegenden Erfindung gewonnen wurde. Signifikant neue unterschiedliche Reflexe bei einer Röntgenbeugung in einem Transmissionsdiffractometer der Firma STOE/Darmstadt sind in Tabelle 1 dargestellt.

**Tabelle 1:**

| **Nr.** | **2 Theta** |
|---|---|
| 1 | 6,7241 |
| 2 | 9,4851 |
| 3 | 11,9034 |
| 4 | 16,3074 |
| 5 | 17,8722 |

- Diffractometer:: Transmission
- Monochromator :: Curved Ge(111)
- Wavelength :: 1.540598 Cu
- Detector :: Curved PSD
- Scan Mode :: Transmission / Stationary PSD / Fixed omega 2Theta scan

Die in Tabelle 1 dargestellten Reflexe besitzen dabei jeweils eine relative Intensität von ≥ 30 % zum Hauptreflex bei 21,2663. Die Toleranz der 2Theta-Werte beträgt maximal ±10 %. Bevorzugt ist eine Abweichung von ±5 %, und ganz besonders bevorzugt liegt die Unsicherheit bei ±1 %. Gewöhnlich treten allerdings bei gut eingestellten und gut geeichten Geräten Fehler von nicht größer als ±0,02 Einheiten auf.

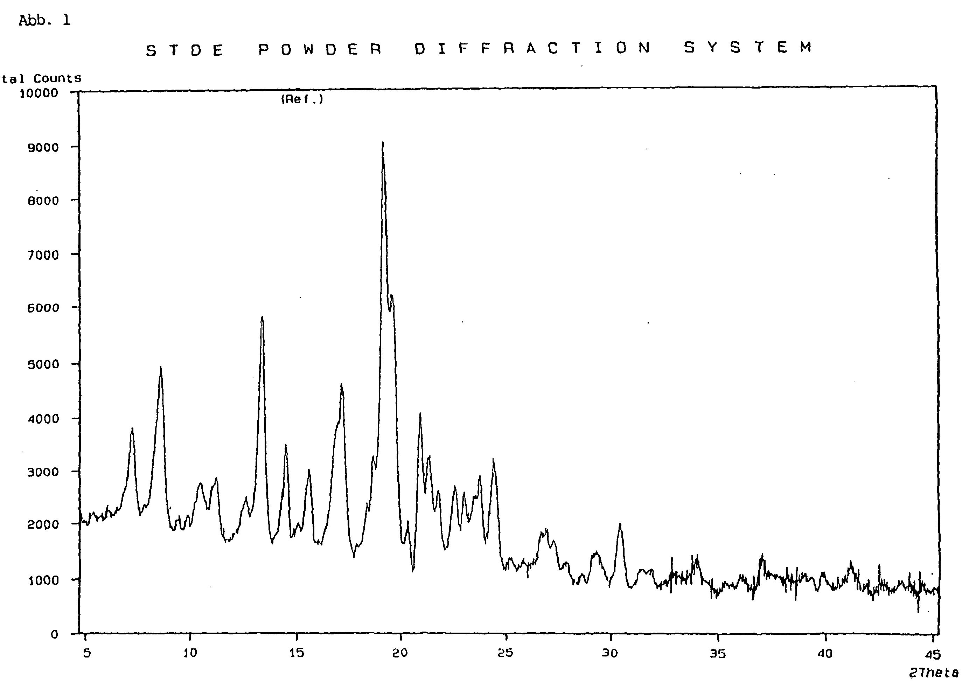

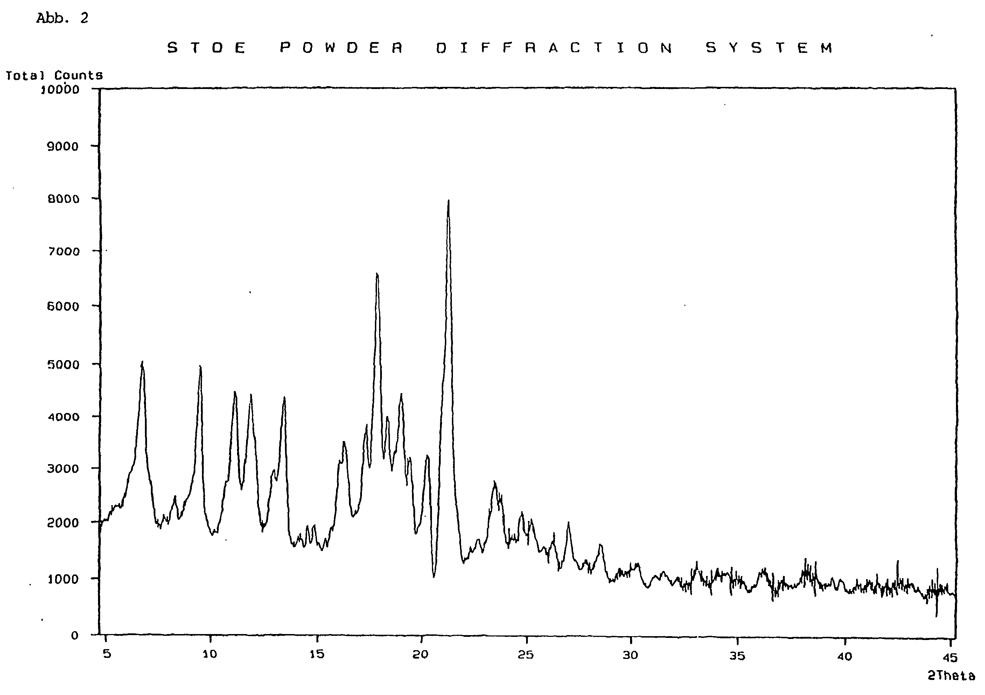

In den Abbildungen 1 und 2 sind die Röntgendiagramme zweier LPE-Proben gegenübergestellt. Abbildung 1 zeigt die Reflexe eines LPEs (I), welches nach dem Stand der Technik hergestellt wurde (Verfahren laut EP 0 719 279, Vergleichsbeispiel 1, vollständig getrocknetes Produkt). Abbildung 2 beinhaltet eine Probe, die nach Beispiel 8 der vorliegenden Erfindung gewonnen wurde. Allerdings zeigen alle Röntgendiagramme der Proben der Beispiele 2-10 gleiche Reflexverteilungen und gleiche relative Intensitäten.

Diese neue Kristallmodifikation führt dazu, daß sich das LPE (I) besonders gut filtrieren läßt.

Erfindungsgemäß liegen die Trocknungszeiten des so hergestellten LPEs (I) deutlich unterhalb von denen des Standes der Technik. Das Auffinden der neuen Kristallmodifikation war damit ursächlich für die Möglichkeit, das LPE-Herstellverfahren ökonomisch vorteilhafter durchführen zu können.

Die folgenden Beispiele sollen die Erfindung verdeutlichen, sie jedoch keinesfalls einschränken.

### Vergleichsbeispiel 1:

### 1-[N²-((S)-Ethoxycarbonyl)-3-phenylpropyl) -N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 120 ml Toluol und 30 ml Ethylacetat wurde der pH-Wert mit konz. Salzsäure auf 1 eingestellt. Man rührte 10 min nach und trennte anschließend die Phasen. Bei 5 °C wurde nun in der wässrigen Phase ein pH-Wert von 4 eingestellt und diese mit 400 ml Ethylacetat extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurde die organische Phase im Vakuum bei max. 33 °C Sumpftemperatur weitgehend eingeengt. Der Sumpf wurde mit 125 ml Toluol versetzt und weiter bis auf 120 g eingeengt. Danach wurden 240 ml Methyl-tert.Butylether zugesetzt und bis auf + 4°C abgekühlt. Dabei entstand ein kristalliner Niederschlag. Zu dieser Kristallsuspension wurden 50 ml Methylcyclohexan bei 4 °C innerhalb von 3 h zugetropft. Es wurde 1 h nachgerührt, filtriert, nachgewaschen und anschließend im Ölpumpenvakuum 4 h bei RT getrocknet.

Ausbeute: 67,7 g ( 85,2 % der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad] |
| 92,0 (+- 0,4) | n. b. | 6400 Toluol 49100 Methyl-tert.- Butylether 770 Methylcyclohexan | -24,0 |
| (n. b. = nicht bestimmt) | | | |

### Beispiel 2:

### 1-[N²-((S)-Ethoxycarbonyl)-3-phenylpropyl)-N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 120 ml Toluol und 30 ml Ethylacetat wurde der pH-Wert mit konz. Salzsäure auf 1 eingestellt. Man rührte 10 min nach und trennte anschließend die Phasen. Bei 40 °C wurde nun in der wässrigen Phase ein pH-Wert von 4 eingestellt und diese mit 450 ml Ethylacetat extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurden zu der organischen Phase 235 ml Methylcyclohexan zugesetzt und im Vakuum bei max. 40 °C Sumpftemperatur bis auf 290 g eingeengt. Der Sumpf wurde auf - 5°C abgekühlt. Dabei entstand ein kristalliner Niederschlag, der filtriert, nachgewaschen und anschließend im Ölpumpenvakuum 4 h bei RT getrocknet wurde.

Ausbeute: 48,0 g (60,5% der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad] |
| 98,3 (+- 0,3 ) | 85 - 90 | n.n. Ethylacetat 126 Methylcyclohexan | -25,2 |
| (n.n. = nicht nachweisbar) | | | |

### Beispiel 3:

### 1-[N²-((S)-Ethoxycarbonyl)-3-phenylpropyl)-N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 120 ml Toluol und 30 ml Ethylacetat wurde der pH-Wert mit konz. Salzsäure auf 1 eingestellt. Man rührte 10 min nach und trennte anschließend die Phasen. Bei 40 °C wurde nun in der wässrigen Phase ein pH-Wert von 4 eingestellt und diese mit 450 ml Ethylpropionat extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurden zu der organischen Phase 225 ml Methylcyclohexan zugesetzt und im Vakuum bei max. 40 °C Sumpftemperatur bis auf 270 g eingeengt. Der Sumpf wurde mit 250 ml Methylcyclohexan versetzt und auf + 5°C abgekühlt. Dabei entstand ein kristalliner Niederschlag, der filtriert, nachgewaschen und anschließend im Ölpumpenvakuum 4 h bei RT getrocknet wurde.

Ausbeute: 59,9 g (75,4% der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad] |
| 96,8 (+- 0,8 ) | 87 - 90 | < 20 Ethylpropionat 598 Methylcyclohexan | -25,5 |

### Beispiel 4:

### 1-[N²-((S)-Ethoxycarbonyl)-3-phenylpropyl)-N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 120 ml Toluol und 30 ml Ethylacetat wurde der pH-Wert mit konz. Salzsäure auf 1 eingestellt. Man rührte 10 min nach und trennte anschließend die Phasen. Bei 40 °C wurde nun in der wässrigen Phase ein pH-Wert von 4 eingestellt und diese mit 450 ml n-Propylacetat extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurden zu der organischen Phase 300 ml n-Hexan zugesetzt und im Vakuum bei max. 40 °C Sumpftemperatur bis auf 240 g eingeengt. Der Sumpf wurde mit 206 ml n-Hexan versetzt und auf + 5°C abgekühlt. Dabei entstand ein kristalliner Niederschlag, der filtriert, nachgewaschen und anschließend im Ölpumpenvakuum 4 h bei RT getrocknet wurde.

Ausbeute: 54,8 g (69,0 % der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad] |
| 97,0 (+- 0,6 ) | 87 - 91 | < 20 n-Propylacetat 43 n-Hexan | -25,4 |

### Beispiel 5:

### 1-[N²-((S)-Ethoxycarbonyl)-3-phenylpropyl)-N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 120 ml Toluol und 30 ml Ethylacetat wurde der pH-Wert mit konz. Salzsäure auf 1 eingestellt. Man rührte 10 min nach und trennte anschließend die Phasen. Bei 40 °C wurde nun in der wässrigen Phase ein pH-Wert von 4 eingestellt und diese mit 450 ml Ethylpropionat extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurde die organische Phase im Vakuum bei max. 40 °C Sumpftemperatur bis auf 230 g eingeengt. Der Sumpf wurde mit 275 ml Cyclohexan versetzt und auf + 5°C abgekühlt. Dabei entstand ein kristalliner Niederschlag, der filtriert, nachgewaschen und anschließend im Ölpumpenvakuum 4 h bei RT getrocknet wurde.

Ausbeute: 60,0 g (75,6% der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad] |
| 97,4 (+- 0,8 ) | 87 - 91 | < 20 Ethylpropionat 524 Cyclohexan | -25,3 |

### Beispiel 6:

### 1-[N²-((S)-Ethoxycarbonyl)-3-phenylpropyl)-N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 120 ml Toluol und 30 ml Ethylacetat wurde der pH-Wert mit konz. Salzsäure auf 1 eingestellt. Man rührte 10 min nach und trennte anschließend die Phasen. Bei 40 °C wurde nun in der wässrigen Phase ein pH-Wert von 4 eingestellt und diese mit 450 ml n-Propylacetat extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurde die organische Phase im Vakuum bei max. 40 °C Sumpftemperatur bis auf 200 g eingeengt. Der Sumpf wurde mit 270 ml Cyclohexan versetzt und auf + 5°C abgekühlt. Dabei entstand ein kristalliner Niederschlag, der filtriert, nachgewaschen und anschließend im Ölpumpenvakuum 4 h bei RT getrocknet wurde.

Ausbeute: 56,5 g (71,2 % der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad] |
| 97,6 (+- 0,2) | 87 - 91 | < 20 n-Propylacetat 253 Cyclohexan | -25,4 |

### Beispiel 7:

### 1-[N²-((S)-Ethoxycarbonyl)-3-phenylpropyl)-N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 120 ml Toluol und 30 ml Ethylacetat wurde der pH-Wert mit konz. Salzsäure auf 1 eingestellt. Man rührte 10 min nach und trennte anschließend die Phasen. Bei 40 °C wurde nun in der wässrigen Phase ein pH-Wert von 4 eingestellt und diese mit 450 ml Methylisobutylketon extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurde die organische Phase im Vakuum bei max. 40 °C Sumpftemperatur bis auf 230 g eingeengt. Der Sumpf wurde mit 185 ml Methylcyclohexan versetzt und auf + 5°C abgekühlt. Dabei entstand ein kristalliner Niederschlag, der filtriert, nachgewaschen und anschließend im Ölpumpenvakuum 4 h bei RT getrocknet wurde.

Ausbeute: 56,1 g (70,6% der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad] |
| 97,6 (+- 0,6) | 87 - 91 | 91 Methylisobutylketon 253 Methylcyclohexan | -25,5 |

### Beispiel 8:

### 1- [N²-((S)-Ethoxycarbonyl)-3-phenylpropyl)-N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 120 ml Toluol und 30 ml Ethylacetat wurde der pH-Wert mit konz. Salzsäure auf 1 eingestellt. Man rührte 10 min nach und trennte anschließend die Phasen. Bei 40 °C wurde nun in der wässrigen Phase ein pH-Wert von 4 eingestellt und diese mit 450 ml Methylisobutylketon extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurde die organische Phase im Vakuum bei max. 40 °C Sumpftemperatur bis auf 185 g eingeengt. Der Sumpf wurde mit 235 ml Cyclohexan versetzt und auf + 5°C abgekühlt. Dabei entstand ein kristalliner Niederschlag, der filtriert, nachgewaschen und anschließend im Ölpumpenvakuum 4 h bei RT getrocknet wurde.

Ausbeute: 60,5 g (76,2% der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad] |
| 98,4 (+- 0,5) | 87 - 91 | 207 Methylisobutylketon 226 Cyclohexan | -25,4 |

### Beispiel 9:

### 1-[N²-((S)-Ethoxycarbonyl)-3-phenylpropyl)-N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 45 ml Methylisobutylketon und 45 ml Cyclohexan wurde der pH-Wert mit konz. Salzsäure auf 1 eingestellt. Man rührte 10 min nach und trennte anschließend die Phasen. Bei 40 °C wurde nun in der wässrigen Phase ein pH-Wert von 4 eingestellt und diese mit 450 ml Methylisobutylketon extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurde die organische Phase im Vakuum bei max. 40 °C Sumpftemperatur bis auf 185 g eingeengt. Der Sumpf wurde mit 235 ml Cyclohexan versetzt und auf + 5°C abgekühlt. Dabei entstand ein kristalliner Niederschlag, der filtriert, nachgewaschen und anschließend im Ölpumpenvakuum 4 h bei RT getrocknet wurde.

Ausbeute: 61 g (76,8 % der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad] |
| 98,6 (+- 0,4) | 87 - 91 | 215 Methylisobutylketon 280 Cyclohexan | -25,6 |

### Beispiel 10:

### 1-[N²-((S)-Ethoxycarbonyl)-3-phenylpropyl)-N⁶-trifluoracetyl]-L-lysyl-L-prolin (Verbindung I)

Es wird eine reduktive Aminierung gemäß Patentanmeldung EP 05 23 449 analog Beispiel 3 Seite 10 durchgeführt.

### Aufarbeitung:

Die Reaktionslösung, deren Einsatzmenge für die Aufarbeitung so gewählt wurde, daß sich darin neben dem typischen Nebenproduktprofil insgesamt 150 mmol des gewünschten LPE-(SSS)-Diastereomeren (I) befanden, wurde im Vakuum bei 45 °C Badtemperatur weitgehend eingedampft. Der Rückstand wurde in 1400 ml Wasser aufgenommen und im Vakuum kurz angestrippt. Nach Zusatz von 45 ml Methylisobutylketon und 45 ml Cyclohexan wurde der pH-Wert mit konz. Salzsäure auf 1 eingestellt. Man rührte 10 min nach und trennte anschließend die Phasen. Bei 40 °C wurde nun in der wässrigen Phase ein pH-Wert von 4 eingestellt und diese mit 450 ml Methylisobutylketon extrahiert. Die Phasen wurden getrennt, die organische Phase mit 110 ml Wasser versetzt und mit Natronlauge (50 %ig) auf einen pH-Wert von 5,8 eingestellt. Nach Phasentrennung wurde die organische Phase im Vakuum bei max. 40 °C Sumpftemperatur bis auf 160 g eingeengt. Der Sumpf wurde mit 175 ml Cyclohexan versetzt und auf + 5°C abgekühlt. Dabei entstand ein kristalliner Niederschlag. Zu dieser Kristallsuspension wurden 103 ml Cyclohexan bei 5 °C innerhalb von 1,5 h zugetropft. Es wurde 1 h nachgerührt, filtriert, nachgewaschen und anschließend im Ölpumpenvakuum 4 h bei RT getrocknet.

Ausbeute: 66,0 g (83,1% der Theorie)

| Analytik: | | | |
|---|---|---|---|
| Gehalt SSS-Diastereomer [Gew%] | Schmelzpunkt [°C] | Restlösungsmittel [mg/kg] | [∝] 25/D (c = 1 MeOH/ 0,1N HCl) [Grad ] |
| 97,4 (+- 0,3) | 87 - 91 | 167 Methylisobutylketon 218 Cyclohexan | -25,6 |

## Patentansprüche

1. Verfahren zur Isolierung von LPE der Formel I 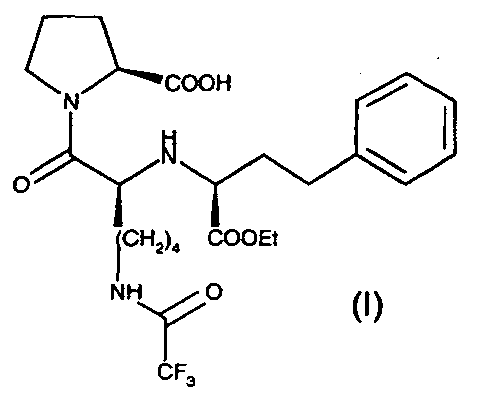 aus einer wäßrigen Lösung des LPE-Herstellprozesses durch Extraktion und anschließende Kristallisation,
**dadurch gekennzeichnet,**
**daß** das zur Extraktion des LPEs (I) benutzte Lösungsmittel oder Lösungsmittelgemisch ebenfalls ein Hauptbestandteil des Lösungsmittels oder Lösungsmittelgemisches ist, welches zur Kristallisation eingesetzt wird und daß man Lösungsmittel oder Lösungsmittelgemische bestehend aus Estern und/oder Ketonen der allgemeinen Formel III 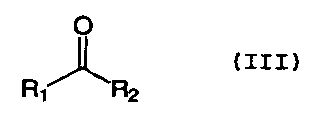 mit
R₁ = (C₁-C₆)-Alkyl und
R₂ = (C₁-C₆)-Alkyl, (C₁-C₆)-Alkoxy,
gegebenenfalls mit offenkettigen aliphatischen oder cycloaliphatischen Kohlenwasserstoffen als Lösungsmittel vermischt, benutzt, wobei man die Kohlenwasserstoffe vor oder nach der Extraktion dem Lösungsmittel oder Lösungsmittelgemisch beifügt.

2. Verfahren nach einem der vorhergehenden Ansprüche,
**dadurch gekennzeichnet,**
**daß** man vor der Extraktion die wäßrige Produktphase bei einem pH zwischen 0 und 3,5 mit einem der Lösungsmittel oder Lösungsmittelgemische behandelt und anschließend die wäßrige Produktphase abtrennt.

3. Verfahren nach einem der vorhergehenden Ansprüche,
**dadurch gekennzeichnet,**
**daß** man vor der Extraktion des LPEs (I) in die organische Phase und nach der Behandlung der wäßrigen Produktphase mit einem der Lösungsmittel oder Lösungsmittelgemische eine Aktivkohleklärung in einem pH-Bereich von 0 bis 3,5 durchführt.

4. Verfahren nach einem der vorhergehenden Ansprüche,
**dadurch gekennzeichnet,**
**daß** man die Extraktion des LPEs (I) aus seiner wäßrigen Lösung bei einem pH-Wert zwischen 3,9 und 4,8 durchführt.

5. Verfahren nach einem der vorhergehenden Ansprüche,
**dadurch gekennzeichnet,**
**daß** man die organische LPE-Extraktionslösung vor der Kristallisation mit Wasser bei einem pH von 4,8 bis 6,3, besonders bevorzugt ist der Bereich von 5,7 bis 6,0, auswäscht und die wäßrige Phase abtrennt.

6. Verfahren nach einem der vorhergehenden Ansprüche,
**dadurch gekennzeichnet,**
**daß** man die LPE-Extraktionslösung vor der Kristallisation durch Destillation azeotrop entwässert.

7. Verfahren nach Anspruch 4 und 5,
**dadurch gekennzeichnet,**
**daß** man die Extraktionsschritte bei Temperaturen zwischen 0 °C und 60 °C durchführt.

8. Verfahren nach einem der vorhergehenden Ansprüche,
**dadurch gekennzeichnet,**
**daß** das Volumenverhältnis im Lösungsmittelgemisch zwischen dem eingesetzten Ester und/oder Keton und dem eingesetzten aliphatischen/cycloaliphatischen Kohlenwasserstoff zwischen 1 : 0,01 und 1 : 100 liegt.

9. Verfahren nach Anspruch 1,
**dadurch gekennzeichnet,**
**daß** die Temperatur bei der Kristallisation zwischen - 40 °C und +50 °C liegt.

10. Verfahren nach Anspruch 7,
**dadurch gekennzeichnet,**
**daß** man nach der Kristallisation erneut zur Mutterlauge ggf. nach deren Einengen einen aliphatischen oder cycloaliphatischen Kohlenwasserstoff zur Vervollständigung der Kristallisation zufügt.

11. LPE mit Reflexen bei
6,7241
9,4851
11,9034
16,3074
17,8722 (2 Theta),
wobei bei 21,2663 der Hauptreflex ist.

## Claims

1. Process for isolating LPE of Formula I 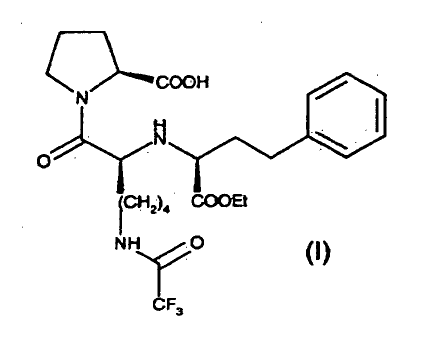 from an aqueous solution from the LPE production process by extraction and subsequent crystallisation, **characterised in that** the solvent or solvent mixture used to extract the LPE (I) is also a main constituent of the solvent or solvent mixture used for crystallisation and **in that** solvents or solvent mixtures consisting of esters and/or ketones of general Formula III 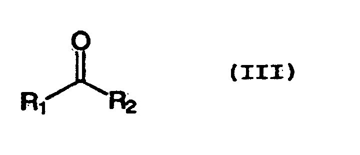 wherein
R₁ = (C₁-C₆)-alkyl and
R₂ = (C₁-C₆)-alkyl, (C₁-C₆)-alkoxy,
are used, optionally mixed with open-chain aliphatic or cycloaliphatic hydrocarbons as the solvent, the hydrocarbons being added to the solvent or solvent mixture prior to or after extraction.

2. Process according to any of the preceding claims, **characterised in that** the aqueous product phase is treated with one of the solvents or solvent mixtures at a pH between 0 and 3.5 prior to extraction and the aqueous product phase is then separated off.

3. Process according to any of the preceding claims, **characterised in that** activated carbon purification is carried out in a pH range of 0 to 3.5 prior to extraction of the LPE (I) into the organic phase and after treatment of the aqueous product phase with one of the solvents or solvent mixtures.

4. Process according to any of the preceding claims, **characterised in that** the LPE (I) is extracted from its aqueous solution at a pH between 3.9 and 4.8.

5. Process according to any of the preceding claims, **characterised in that** the organic LPE extraction solution is rinsed prior to crystallisation with water at a pH of 4.8 to 6.3, the range of 5.7 to 6.0 being particularly preferred, and the aqueous phase is separated off.

6. Process according to any of the preceding claims, **characterised in that** the LPE extraction solution is dewatered azeotropically by distillation prior to crystallisation.

7. Process according to claim 4 and 5, **characterised in that** the extraction steps are carried out at temperatures between 0°C and 60°C.

8. Process according to any of the preceding claims, **characterised in that** the volume ratio in the solvent mixture between the ester and/or ketone used and the aliphatic/cycloaliphatic hydrocarbon used is between 1:0.01 and 1:100.

9. Process according to claim 1, **characterised in that** the temperature during crystallisation is between -40°C and +50°C.

10. Process according to claim 7, **characterised in that**, after crystallisation, an aliphatic or cycloaliphatic hydrocarbon is again added to the mother liquor, optionally once it has been concentrated, to complete crystallisation.

11. LPE with reflexes at
6,7241
9,4851
11,9034
16,3074
17,8722 (2 Theta),
wherein the main reflex is at 21.2663.

## Revendications

1. Procédé pour l'isolement de LPE de formule 1 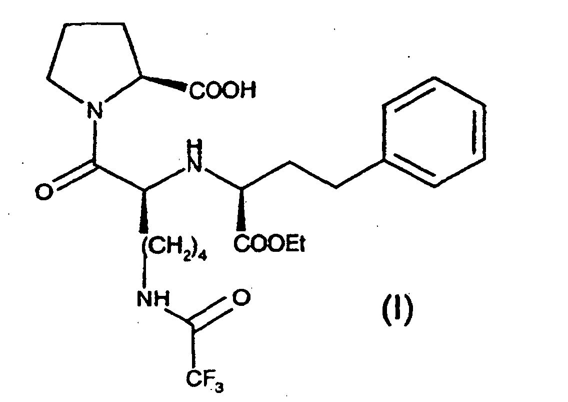 à partir d'une solution aqueuse provenant du processus de préparation de LPE par extraction et cristallisation consécutive, **caractérisé en ce que** le solvant ou le mélange de solvants utilisé pour l'extraction du LPE (I) est également un constituant principal du solvant ou du mélange de solvants utilisé pour la cristallisation et **en ce qu'**on utilise des solvants ou des mélanges de solvants qui sont constitués d'esters et/ou de cétones de formule générale III 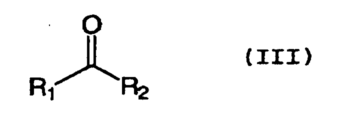 avec
R₁ = alkyle en C₁ à C₆ et
R₂ = alkyle en C₁ à C₆, alcoxy en C₁ à C₆,
le cas échéant mélangés avec des hydrocarbures aliphatiques à chaîne ouverte ou cycloaliphatiques comme solvants, les hydrocarbures étant ajoutés avant ou après l'extraction au solvant ou mélange de solvants.

2. Procédé selon l'une quelconque des revendications précédentes, **caractérisé en ce qu'**on traite avant l'extraction la phase aqueuse de produit à un pH entre 0 et 3,5 avec un des solvants ou mélanges de solvants, puis on sépare la phase aqueuse de produit.

3. Procédé selon l'une quelconque des revendications précédentes, **caractérisé en ce qu'**on effectue un éclaircissement au charbon actif dans une plage de pH de 0 à 3,5 avant l'extraction du LPE (I) dans la phase organique et après le traitement de la phase aqueuse de produit avec un des solvants ou mélanges de solvants.

4. Procédé selon l'une quelconque des revendications précédentes, **caractérisé en ce qu'**on effectue l'extraction du LPE (I) à partir de sa solution aqueuse à un pH entre 3,9 et 4,8.

5. Procédé selon l'une quelconque des revendications précédentes, **caractérisé en ce qu'**on lave la solution d'extraction organique du LPE avant la cristallisation avec de l'eau à un pH de 4,8 à 6,3, la plage de 5,7 à 6,0 étant particulièrement préférée, puis on sépare la phase aqueuse.

6. Procédé selon l'une quelconque des revendications précédentes, **caractérisé en ce qu'**on déshydrate la solution d'extraction du LPE avant la cristallisation par distillation d'un azéotrope.

7. Procédé selon les revendications 4 et 5, **caractérisé en ce qu'**on réalise les étapes d'extraction à des températures entre 0°C et 60°C.

8. Procédé selon l'une quelconque des revendications précédentes, **caractérisé en ce que** le rapport volumique dans le mélange de solvants entre l'ester utilisé et/ou la cétone utilisée et l'hydrocarbure aliphatique/cycloaliphatique utilisé est compris entre 1:0,01 et 1:100.

9. Procédé selon la revendication 1, **caractérisé en ce que** la température lors de la cristallisation est située entre -40°C et +50°C.

10. Procédé selon la revendication 7, **caractérisé en ce qu'**on ajoute à nouveau un hydrocarbure aliphatique ou cycloaliphatique après la cristallisation à la lessive-mère, le cas échéant après sa concentration, afin de compléter la cristallisation.

11. LPE avec des réflexions à
6,7241
9,4851
11,9034
16,3074
17,8722 (2 thêta)
la réflexion principale étant située à 21,2663.
